# EUROPÄISCHE PATENTSCHRIFT

(11) **EP 0 894 525 B1**
(45) Veröffentlichungstag und Bekanntmachung des Hinweises auf die Patenterteilung: **09.04.2003**
(21) Anmeldenummer: 98113874.6
(22) Anmeldetag: 24.07.1998
(51) Int. Cl.: B01F 7/00

(54) **Hubvorrichtung für ein Aggregat, insbesondere ein Tauchmotorrührgerät in einem dichten Behälter**
Hoisting apparatus for an aggregate, in particular a submersible motorised stirrer in a closed vessel
Dispositif de soulèvement pour un aggregat, en particulier un agitateur submersible motorisé dans une cuve hermétique

(30) Priorität: 26.07.1997 DE 19732198
(43) Veröffentlichungstag der Anmeldung: 03.02.1999
(73) Patentinhaber: U.T.S. Umwelt-Technik-Süd GmbH, 84419 Obertaufkirchen (DE)
(72) Erfinder: Bürger Adam, 84405 Grüntegernbach (DE)
(74) Vertreter: Neubauer, Hans-Jürgen, Dipl.-Phys.

(56) Entgegenhaltungen:
- DE-A- 4 120 987
- DE-C- 19 517 901
- DE-U- 9 414 920
- DE-U- 29 502 974
- US-A- 4 721 392

## Beschreibung

Die Erfindung betrifft eine Hubvorrichtung für Aggregate, insbesondere ein Tauchmotorrührgerät, in einem dichten Behälter, insbesondere in einem Fermenterbehälter einer Biogasanlage nach dem Oberbegriff des Anspruchs 1.

In Biogasanlagen läuft ein Fermentationsprozeß ab, bei dem organische Stoffe, wie z. B. Gras, Stalldung, Jauche, Klärschlamm, Stroh und dergleichen vergast werden. Diese Gase sammeln sich in einem oberen Fermenterbehälterbereich und können direkt zur Energieerzeugung verwendet werden, z. B. als Heizgas zur Stromerzeugung in nachgeschalteten Brennkraftmaschinen mit Elektrogeneratoren. Der Fermentationsprozeß findet in einem Fermenterbehälter statt, in dem die organischen Stoffe mit Flüssigkeit versetzt werden und der Fermentations- bzw. der Vergasungsprozeß unter aeroben oder anaeroben Bedingungen durch Mikroorganismen, wie z. B. Hefen, Bakterien, etc. durchgeführt wird. Zur Verteilung der organischen Feststoffe in der flüssigen Phase ist ein Tauchmotorrührgerät bekannt, das mittels einer Seilwinde einer Hubvorrichtung entlang eines Aggregatträgers verfahrbar ist.

Bei bekannten Hubvorrichtungen wird davon ausgegangen, daß die Seiltrommel der Seilwinde außerhalb des Behälters im Bereich oberhalb einer oberen Behälterwand anzuordnen ist. Dies erfordert allerdings eine komplizierte und problembehaftete gasdichte Seildurchführung durch die Behälterwand.

Eine aus der DE 41 20 987 A1 bekannte, gattungsgemäße Hubvorrichtung umfaßt jeweils einen vertikal im Behälter angeordneten Aggregatträger, der in oberen und unteren Lagern gelagert ist und an dem das Aggregat höhenverstellbar gehalten ist. Die Hubvorrichtung weist jeweils eine Höhenverstelleinrichtung als Seilwinde für das Aggregat auf, die eine mit dem Aggregatträger fest verbundene Seiltrommel mit horizontaler Seiltrommelwelle, ein einerseits mit dem Aggregat verbundenes und andererseits auf die Seiltrommel aufwickelbares Seil, einen Seiltrommelantrieb und eine außerhalb des Behälters angeordnete Betätigungseinrichtung für den Seiltrommelantrieb umfaßt.

Auch hier ist die Seiltrommel samt Kurbel als Betätigungseinrichtung oberhalb einer oberen Behälterwand und damit außerhalb des Behälters an einem Aggregatträger angeordnet. Zur gasdichten Seildurchführung durch die obere Behälterwand ist das Seil von der Seiltrommel ausgehend innerhalb des hohlen Aggregatträgers in den Flüssigkeitsbereich nach unten geführt. Dort tritt das Seil durch eine Austrittsöffnung hindurch aus dem Aggregatträger aus und wird über eine Umlenkrolle umgelenkt außerhalb des Aggregaträgers nach oben zu einem oberen Bereich des Aggregatträgers geführt, wobei die Gasdichtheit durch die über der Austrittsöffnung liegende Flüssigkeit erhalten wird. Dort wird das Seil dann über weitere Umlenkrollen wiederum umgelenkt und nach unten zu einem Tauchmotorrührgerät geführt.

Ein wesentlicher Nachteil eines derartigen Aufbaus ist, daß sich die Austrittsöffnung des Seils am unteren Trägerende insbesondere mit langfasrigen Feststoffen zusetzen und dadurch die Seilführungsrolle blockieren kann, so daß eine Höhenverstellung des Aggregats nicht mehr möglich ist. Ebenso ist bei einem derartigen Aufbau eine lange Seilführung mit mehreren Umlenkrollen notwendig.

Aus der DE 295 02 974 U1 ist eine Hubvorrichtung für eine Biogasanlage bekannt, bei der die Seiltrommel samt Betätigungseinrichtung ebenfalls außerhalb des Behälterdeckels an einem Aggregatträger angeordnet ist. Zur gasdichten Seildurchführung ist das Seil hier außen an dem Aggregatträger entlang bis in eine flüssigkeitsbefüllte Siphondichtung geführt. Dort wird das Seil über eine erste Umlenkrolle um das innere, mit der Behälterdecke verbundene Siphonrohr nach oben und schließlich über eine zweite Umlenkrolle um das äußere, mit dem Aggregatträger verbundene und nach unten abgeschlossene Siphonrohr nach unten zu einem Tauchmotorrührgerät geführt.

Eine derartige, in der Regel mit Wasser befüllte Siphondichtung weist den Nachteil auf, daß das Wasser in der Dichtung insbesondere bei höheren Umgebungstemperaturen schnell verdunstet, so daß die Dichtwirkung dann nicht mehr gegeben ist. Bei sehr tiefen Temperaturen kann das Wasser dagegen frieren und damit die Umlenkrollen sowie die Seilführung blockieren. Deshalb ist mit einem derartigen Aufbau eine ständige, aufwendige Kontrolle notwendig. Weiter ist zur Lagerung des oberen Aggregatträgerendes eine zusätzliche Lagerkonsole im Deckelbereich vorzusehen, da aufgrund der Siphondichtung und des durch die Siphondichtung geführten Seils die Lagerung des Aggregatträgers in diesem Bereich nicht stabil durchzuführen ist. Ebenso kann mit einem derartigen Aufbau keine Verschwenkung des Aggregatträgers um dessen Längsachse realisiert werden, sondern lediglich eine Höhenverstellung des Aggregats.

Schließlich ist auch eine Hubvorrichtung für eine Biogasanlage aus der DE 195 17 901 C1 bekannt. Auch hier ist eine außerhalb des Behälters an einem Aggregatträger angeordnete Seiltrommel vorgesehen, von der ausgehend das Seil durch eine Stopfbuchsendichtung in die Behälter geführt ist. Die Stopfbuchse ist zweiteilig aus Gewinderohren aufgebaut und auf einem Drehteller angeordnet, der sich, um eine Verschwenkung des Aggregatträgers um dessen Längsachse zu ermöglichen, über Gleitringe mitdreht. Insgesamt handelt es sich hierbei um eine komplizierte und aufwendige Seildurchführung sowie Lagerung des Aggregatträgers im Bereich einer Behälterdecke. Probleme ergeben sich hier insbesondere dadurch, daß das verwendete Stahlseil mit der Zeit auffranst und dadurch eine scharfe Schneidkante ausbildet, die im Rahmen der Auf- und Abbewegung die Stopfbuchse beschädigt. Die Stopfbuchse bildet dann ein Leck, durch das das Biogas in die Umgebung entweichen kann. Dies erhöht aufgrund der durchzuführenden Wartungsarbeiten die Reparaturkosten erheblich.

Aufgabe der Erfindung ist es daher, eine Hubvorrichtung für Aggregate, insbesondere ein Tauchmotorrührgerät in einem dichten Behälter, insbesondere in einem Fermenterbehälter einer Biogasanlage zu schaffen, die einen betriebssicheren, montage- und wartungsfreundlichen, einfach zu bedienenden und dichten Aufbau mit einfacher Seilführung aufweist.

Diese Aufgabe wird mit den Merkmalen des Anspruchs 1 gelöst.

Zweckmäßige weite bildengen der Erfindung sind begenstand der abhängëgen Ansprüche 2 bis 16.

Gemäß Anspruch 1 ist die Seiltrommel innerhalb im oberen Bereich des Behälters angeordnet, so daß keine gasdichte Seildurchführung durch eine Behälterwand erforderlich ist. Dabei ist die außerhalb des Behälters angeordnete Betätigungseinrichtung mit dem mittels einer dichten Durchführung durch eine Behälterwand geführten Seiltrommelantrieb verbunden.

Durch diesen zweiteiligen Aufbau, bei dem ein Teil außerhalb des Behälters und ein Teil innerhalb des Behälters liegt, ist keine problembehaftete gasdichte Seildurchführung durch eine Behälterwand erforderlich, sondern lediglich eine unproblematische dichte Durchführung für den Seiltrommelantrieb, die mit an sich bekannten Mitteln einfach und dauerhaft dicht ohne Wartungsaufwand herstellbar ist.

Bei einem derartigen Aufbau der Hubvorrichtung mit innenliegender Seiltrommel ist eine hohe Betriebssicherheit gewährleistet, da lediglich eine einfache Seilführung direkt von der Seiltrommel zum Aggregat ohne aufwendige und komplizierte Umlenkungen über störungsanfällige Umlenkrollen erforderlich ist.

Die Betätigung der Seiltrommel mit der Betätigungseinrichtung erfolgt auf einfache Weise von der Behälteraußenseite her, wobei die dort zugeschaltete Energiezufuhr oder aufgebrachte Bewegungsenergie über den Seiltrommelantrieb auf die Seiltrommel übertragen wird.

Die erfindungsgemäße Anwendung wird bevorzugt in Biogasanlagen in Verbindung mit Tauchmotorrührgeräten verwendet. Die Anordnung kann vorteilhaft auch bei anderen, ähnlichen Anwendungsfällen ggf. mit anderen Aggregaten wie z.B. Tauchmotorpumpen verwendet werden.

Gemäß Anspruch 2 umfaßt der Behälter einen Dom, wie er z.B. aus der Patentanmeldung P 197 14 342.3-41 bekannt ist. Der Dom ist auf eine obere Behälterwand dicht aufgesetzt, wobei ein oberer Bereich des Aggregatträgers, an dem die Seiltrommel angeordnet ist, in den Dom ragt. Dieser Dom weist wenigstens eine gasdicht verschließbare Domöffnung auf, durch die hindurch ein Zugang zu einem mittels der Höhenverstelleinrichtung aus dem Behälterinnenraum in den Dominnenraum bewegten Aggregat möglich ist. Damit brauchen sowohl einfache als auch umfangreiche Reparaturund/oder Wartungsarbeiten vom Monteur nicht mehr im Behälterinnenraum durchgeführt zu werden, sondern können bequemer und einfacher von außen durch die Domöffnung durchgeführt werden. Zum Beispiel bei einem Fermenterbehälter braucht dann der Fermentationsprozeß bei Reparatur- und/oder Wartungsarbeiten nicht mehr unwirtschaftlich zurückgefahren zu werden, sondern kann kontinuierlich fortlaufen. Die gesamte Anlage ist somit wirtschaftlicher zu betreiben.

Der Dom kann jede erdenkliche Form aufweisen, z. B. einen runden oder eckigen Grundriß aufweisen oder zylinderförmig bzw. kastenförmig aufgebaut sein. Die Abmessungen des Doms sind ebenfalls je nach Anwendungsfall beliebig wählbar, so z. B. mannshoch ausführbar. Insbesondere kann auch ein Fenster in einer Domwand vorgesehen sein, damit die Vorgänge im Inneren von Außen ohne Öffnen des Doms beobachtet werden können.

In einer nach Anspruch 3 besonders bevorzugten Ausführungsform umfaßt die Hubvorrichtung eine Drehverstelleinrichtung für den Aggregatträger mit einem Drehantrieb, mit der dieser um seine vertikale Längsachse verdrehbar in einer bestimmten Drehstellung einstellbar und festlegbar ist, wobei das Aggregat an dem Aggregatträger mitdrehend gehalten ist. Eine derartige Verdrehung des Aggregatträgers um dessen Längsachse ist aufgrund der innenliegenden Seiltrommel ohne weiteres möglich, da keine komplizierte, gasdichte Seildurchführung durch eine Behälterwand mehr erforderlich ist. Mit einer derartigen Drehverstellung des Aggregatträgers kann das Aggregat zusätzlich zur vertikalen Positionierung durch die Höhenverstelleinrichtung auch in einer senkrecht zur Aggregatträgerlängsachse liegenden Horizontalebene verschwenkt werden. Damit ist eine stets richtige Positionierung des Aggregats gewährleistet.

Gemäß Anspruch 4 ist der Drehantrieb der Drehverstelleinrichtung ein bevorzugt im oberen Bereich des Aggregatträgers innerhalb des Behälters angebrachter Elektromotor, der eine Motordrehbewegung bevorzugt über eine Zahnradanordnung auf den Aggregatträger überträgt und der von außerhalb des Behälters über eine Steuereinheit als Betätigungseinrichtung, bevorzugt über einen Links-/Rechtslauf-Taster, ansteuerbar und über eine dichte Kabeldurchführung durch eine Behälterwand, bevorzugt eine Domwand mit der Steuereinheit verbunden ist. Ein derartiger Drehantrieb ist einfach zu bedienen. Als Elektromotor kann ein handelsüblicher, gasdicht gekapselter und explosionsgeschützter Elektromotor verwendet werden, der eine hohe Betriebssicherheit garantiert. Weiter wird als dichte Kabeldurchführung bevorzugt die ohnehin in einer Behälterwand vorzusehende Kabeldurchführung für den Aggregatantrieb verwendet. Eine derartige dichte Kabeldurchführung ist einfach zu realisieren, so daß dort keine Abdichtungsprobleme auftreten. Die Steuereinheit dient bevorzugt sowohl zur Betätigung des Aggregatantriebs als auch zur Betätigung des Drehantriebs. Die Arretierung in einer bestimmten Schwenkstellung wird durch das selbsthemmende Getriebe des Elektromotors bewirkt.

Alternativ dazu ist gemäß Anspruch 5 eine ortsfeste Drehantriebswelle des Drehantriebs des Aggregatträgers, bevorzugt als Verlängerung des Aggregatträgers, durch eine dichte Wellendurchführung in allgemein bekannten und bewährten Ausführungsformen durch eine Behälterwand, bevorzugt eine Domwand geführt. Ein derartiger Drehantrieb ist einfach und damit preiswert herzustellen sowie wenig störanfällig.

Gemäß Anspruch 6 ist eine Betätigungseinrichtung für den Drehantrieb ein gelenkig mit dem äußeren Ende der ortsfesten Drehantriebswelle verbundener, aufschwenkbarer Stellhebel, der in einer bestimmten Drehstellung über eine Arretiereinrichtung, bevorzugt eine Gabelraste, an einer Behälterwand, bevorzugt einer oberen Domwand, festlegbar ist. Mit einer derartigen Betätigungseinrichtung für den Drehantrieb ist eine kontrollierte und definierte Verschwenkung des Aggregatträgers um dessen Längsachse und damit des Aggregats in einer Horizontalebene im Behälterinnenraum gewährleistet. Die Position des Stellhebels zeigt dabei die jeweilige Schwenkstellung des Aggregats im Behälter an.

Die Festlegung des Stellhebels durch die Arretiereinrichtung dient zur Fixierung des Aggregatträgers in einer bestimmten Schwenkstellung. Anstelle eines gelenkig angebundenen, aufschwenkbaren Stellhebels kann auch eine starre Anbindung des Stellhebels an das äußere Ende der ortsfesten Drehantriebswelle vorgesehen sein. Die Fixierung erfolgt hierbei z. B. über einen Arretierstift, der den Stellhebel z. B. an einer ortsfesten, zugeordneten Stellscheibe festlegt.

Nach Anspruch 7 sind Anschläge zur Begrenzung einer Drehverstellung des Aggregatträgers an dem Aggregatträger und/oder einer Behälterwand, bevorzugt einer Domwand angeordnet. Derartige Anschläge ermöglichen eine definierte Verschwenkung des Aggregats in einer Horizontalebene, wobei diese Anschläge insbesondere verhindern, daß ein Aggregat, falls der Aggregatträger in der Nähe einer Behälterwand angeordnet ist, an der Behälterinnenwand anschlägt. Ebenso kann durch eine derartige definierte Verschwenkung innerhalb eines bestimmten Schwenkbereichs vermieden werden, daß das Aggregatkabel durch unaufmerksame Handhabung mehrmals um den Aggregatträger gewickelt wird, was den Betrieb beeinträchtigen kann.

Gemäß Anspruch 8 umfaßt der Seiltrommelantrieb einen an der Seiltrommel und damit innerhalb des Behälters angebrachten Elektromotor, der von außerhalb des Behälters über eine Steuereinheit als Betätigungseinrichtung, bevorzugt über einen Links-/Rechtslauf-Taster, ansteuerbar und über eine dichte Kabeldurchführung durch eine Behälterwand, bevorzugt eine Domwand, mit der Steuereinheit verbunden ist. Als Elektromotor kann hier ebenfalls ein gasdicht gekapselter, explosionsgeschützter, herkömmlicher Elektromotor verwendet werden. Als Kabeldurchführung kann die ohnehin für den Aggregatmotor im Behälter vorgesehene dichte Kabeldurchführung verwendet werden. Eine derartige dichte Kabeldurchführung ist einfach zu realisieren, so daß dort keine Abdichtungsprobleme auftreten. Um eine Verschwenkung des Aggregatträgers um dessen Längsachse nicht zu behindern, braucht das Kabel nur eine ausreichende Länge aufweisen. Die Steuereinheit dient bevorzugt sowohl zur Betätigung des Aggregatantriebs als auch zur Betätigung des Elektromotors des Seiltrommelantriebs. Somit kann eine betriebssichere Hubvorrichtung realisiert werden, mit der sowohl eine Drehverstellung des Aggregatträgers als auch eine Höhenverstellung des Aggregats jeweils von außerhalb des Behälters möglich ist.

Alternativ dazu ist nach Anspruch 9 eine ortsfeste Welle des Seiltrommelantriebs durch eine dichte Wellendurchführung durch eine Behälterwand, bevorzugt eine seitliche Domwand, geführt. Der Seiltrommelantrieb umfaßt Mittel zur Umlenkung der Bewegungsübertragungsrichtung, wobei die Umlenkung in der Drehachse des Aggregatträgers erfolgt. Die Mittel zur Umlenkung der Bewegungsübertragungsrichtung sind einerseits mit der ortsfesten Welle und andererseits mit der Seiltrommelwelle verbunden. Mit einer derartig aufgebauten Hubvorrichtung ist es somit möglich, sowohl eine Höhenverstellung des Aggregats entlang des Aggragatträgers als auch eine Drehverstellung des Aggregaträgers selbst vorzunehmen. Dabei ist lediglich eine einfach mit bekannten Mitteln und Lagern herzustellende, dichte Wellendurchführung für die ortsfeste Welle durch eine Behälterwand vorzusehen.

Nach Anspruch 10 umfassen die Mittel zur Umlenkung der Bewegungsübertragungsrichtung eine Gelenkwelle, deren wenigstens ein Gelenk in der Drehachse des Aggregatträgers liegt und deren beidseitige Wellenteile die ortsfeste Welle und die Seiltrommelwelle sind. Als Gelenkwellen werden z. B. handelsübliche und preiswerte Einfach-Wellengelenke oder Doppel-Wellengelenke verwendet, die eine Drehbewegung von der ortsfesten Welle auf die Seiltrommelwelle und damit die Seiltrommel übertragen. Als Seiltrommelwelle kann hier auch eine starre Seiltrommelachse dienen, die mit einem Wellenteil der Seiltrommel zusammenwirkt und eben die Seiltrommelwelle bildet. Bei einer Verschwenkung des Aggregatträgers um dessen Längsachse wird zudem die Seiltrommelwelle relativ zu der ortsfesten Welle in einer in etwa vertikal zur Aggregatträgerlängsachse liegenden Horizontalebene verschwenkt. Der Schwenkbereich wird dabei durch die Gelenklagergeometrie vorgegeben. Somit ist hier auf einfache Weise mit einer Gelenkwelle sichergestellt, daß das Aggregat über die Hubvorrichtung sowohl in der Höhe verstellt als auch in einer Horizontalebene verschwenkt werden kann. Die Gelenkwelle ist über die ortsfeste Welle auf einfache Weise z. B. in einer gasdichten Buchse in der Behälterwand gelagert. Dabei dient die Buchse bevorzugt auch als Gelenkwellenführung. Ebenso kann die Gelenkwelle aber auch zusätzlich im Bereich des Wellengelenks in einem Gelenklager geführt sein.

Nach Anspruch 11 umfassen die Mittel zur Umlenkung der Bewegungsübertragungsrichtung einen Profilring, bevorzugt einen geradverzahnten oder kegelradverzahnten Zahnring, der frei drehbar am Aggregatträger angeordnet ist und der mit je einem zugeordneten Eingriffsprofil an der ortsfesten Welle und der Seiltrommelwelle in Eingriff steht. Als Profilring wird bevorzugt ein quer zur Längsachse eines Aggregatträgers auf diesem frei drehbares Tellerrad verwendet werden, der über einen randseitigen, geradverzahnten Zahnring einerseits mit einem geradverzahnten, bevorzugt endseitigen Zahnprofil der ortsfesten Welle des Seiltrommelantriebs und andererseits mit einem geradverzahnten, bevorzugt endseitigen Zahnrad der Seiltrommelwelle in Eingriff steht. Die beiden Wellen liegen bei einem derartigen Aufbau hinsichtlich der Längsachse des Aggregatträgers bevorzugt in einer gleichen Höhe und weisen gleiche Zahnräder auf, so daß sich ein in etwa gestreckter Aufbau der Mittel zur Umlenkung der Bewegungsübertragungsrichtung ergibt.

Bei einer Betätigung der Betätigungseinrichtung wird die Drehbewegung der ortsfesten Welle über deren Zahnrad auf das Tellerrad und von dort auf das Zahnrad der Seiltrommelwelle übertragen, so daß die Seiltrommel je nach der gewählten ursprünglichen Drehrichtung der Betätigungseinrichtung das Aggregat nach oben oder nach unten entlang des Aggregatträgers verfährt. Durch die frei drehbare Profilringanordnung ist es zudem möglich, den Aggregatträger ohne Einfluß auf den Seiltrommelantrieb in einer Ebene quer zu dessen Längsachse zu verschwenken.

Alternativ kann der Profilring an einem in Längsrichtung des Aggregatträgers gesehen oberen und unteren Profilringrand ein umlaufendes Kegelradprofil aufweisen. Mit dem oberen Kegelradprofil des Profilrads kämmt ein bevorzugt endseitiges Kegelrad der ortsfesten Welle des Seiltrommelantriebs, während mit dem unteren Kegelradteil des Profilrings ein ebenfalls bevorzugt endseitiges Kegelrad der Seiltrommelwelle kämmt. Die Bewegungsübertragung funktioniert hier genauso wie mit dem vorher beschriebenen Tellerradaufbau, nur daß die beiden bevorzugt mit gleichen Kegelradprofilen versehenen Wellen des Seiltrommelantriebs hier für einen kompakten Aufbau bevorzugt übereinander angeordnet sind.

Nach Anspruch 12 ist die Betätigungseinrichtung eine Kurbel, die mit der ortsfesten Welle des Seiltrommelantriebs verbunden ist. Eine derartige herkömmliche Kurbel kann von einem Bediener auf einfache Weise mit der Hand betätigt werden, wobei je nach der gewünschten Höhenverstellungsrichtung des Aggregats die Kurbel nach links oder nach rechts gedreht wird. Die Geometrie der Kurbel ist dabei an die entsprechend aufzuwendende Kraft angepaßt.

Nach Anspruch 13 ist an dem Aggregatträger wenigstens ein oberer Anschlag zur definierten Höhenverstellung des Aggregats mit dem Seiltrommelantrieb angeordnet. Ein derartiger oberer Anschlag verhindert ein unkontrolliertes Hochziehen des Aggregats, z. B. zu hoch in den Dominnenraum.

Nach Anspruch 14 ist der Aggregatträger mit einem oberen Trägerende oder einer ggf. mit dem oberen Trägerende verbundenen Drehantriebswelle in einer oberen Behälterwand, bevorzugt einer oberen Domwand und mit einem unteren Trägerende in einer unteren Behälterwand dicht gelagert. Durch die im Inneren angeordnete Seiltrommel läßt sich eine stabile und vor allem einfach auszuführende Lagerung von insbesondere dem oberen Trägerende in einer Behälterwand realisieren, bei der keine Rücksicht auf eine gasdichte Seildurchführung in diesem Bereich genommen werden muß. Als Dichtung kann z. B. eine herkömmliche gasdichte Buchsendichtung verwendet werden.

Nach Anspruch 15 ist der Aggregatträger zweiteilig aus einem unteren Trägerteil und einem oberen Trägerteil mit einer Trennlinie im Bereich unterhalb der festen Anbindung der Seiltrommel an den Aggregatträger aufgebaut, wobei das untere Trägerteil ein Profilträger ist und das obere Trägerteil wenigstens im Bereich einer Behälterwanddurchführung, bevorzugt einer Domwanddurchführung, die Drehantriebswelle des Drehantriebs bildet, die miteinander ggf. lösbar verbunden sind. Mit einer derartigen Aufteilung des Aggregatträgers in oberes und unteres Trägerteil kann bei der Montage eine einfache und schnelle Anpassung an die jeweils vorherrschenden Einbaugegebenheiten erreicht werden. So ist es z. B. möglich, zuerst den unteren Träger in den Behälter einzubringen, diesen auf die richtige Länge abzulängen und dann den oberen Trägerteil zusammen mit z. B. der Domwand aufzusetzen. Das obere und untere Trägerteil können dabei an ihrer Schnittstelle verschweißt werden bzw. auch lösbar miteinander verbunden sein, um z. B. eine schnelle Demontage zu ermöglichen.

Nach Anspruch 16 ist der Aggregatträger wenigstens teilweise als Profilträger mit offenem Profilquerschnitt ausgebildet. Ein derartiger Profilträger ermöglicht es auf einfache Weise sicherzustellen, daß sich das Aggregat bei einer Verdrehung des Aggregatträgers um dessen Längsachse mitdreht. Da der Profilträger nicht hohl ausgebildet sein muß, um z. B. ein Seil für eine gasdichte Seildurchführung aufzunehmen, wie dies im Stand der Technik der Fall ist, kann er mit einem einfachen, offenen Profilquerschnitt ausgebildet sein, der einfacher herzustellen und damit preiswerter als ein geschlossener Profilquerschnitt ist.

Die Erfindung wird nachfolgend anhand einer Zeichnung näher erläutert.

Es zeigen:
- Fig. 1: eine erste Ausführungsform einer Hubwelle mit einem Elektromotor als Seiltrommelantrieb,
- Fig. 2: eine zweite Ausführungsform einer Hubvorrichtung mit einer Gelenkwellenanordnung,
- Fig. 3: eine schematische Draufsicht auf ein Aggregat der in Fig. 2 dargestellten Ausführungsform einer Hubvorrichtung,
- Fig. 4: eine dritte Ausführungsform einer Hubvorrichtung mit einer Tellerradanordnung, und
- Fig. 5: eine vierte Ausführungsform der Hubeinrichtung mit einer Kegelradanordnung.

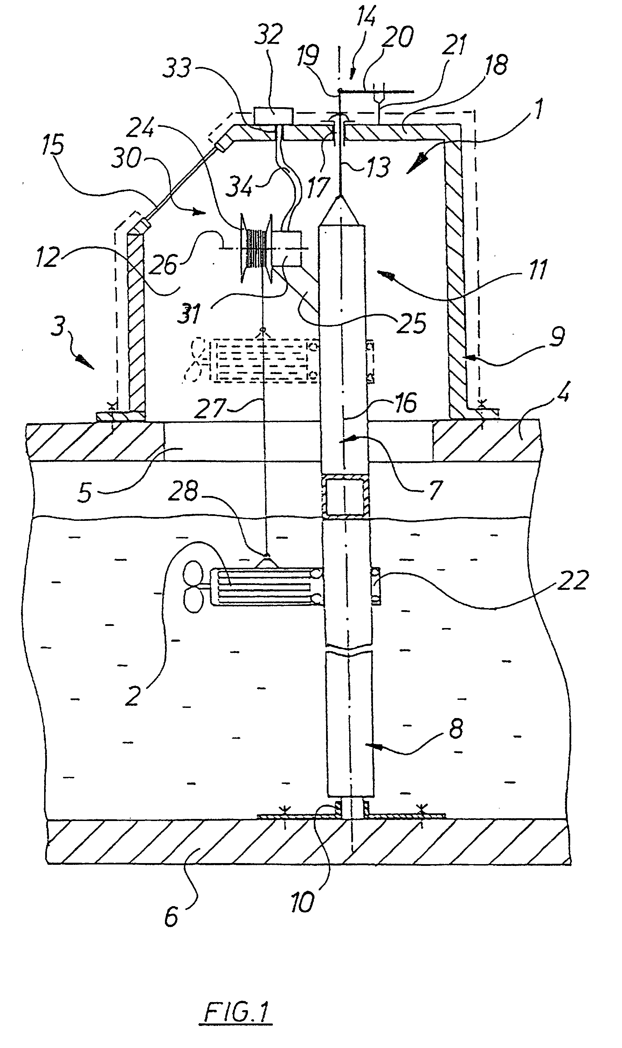

In der Fig. 1 ist eine erste Ausführungsform einer Hubvorrichtung 1 für ein Tauchmotorrührgerät 2 in einem dichten Fermenterbehälter 3 einer Biogasanlage dargestellt. Eine obere Behälterwand 4 umfaßt eine Öffnung 5, um die herum ein Dom 9 dicht auf die obere Behälterwand 4 aufgesetzt ist. Der Fermenterbehälter 3 kann dabei ganz oder wenigstens teilweise in den Boden versenkt sein. Der Dom 9 kann an der Außenseite eine Wärmeisolierung, wie die in der Fig. 1 strichliert dargestellt ist, und ferner ein Kontrollfenster 15 aufweisen.

Ein Aggregatträger 7 ist vertikal im Fermenterbehälter 3 angeordnet. Der Aggregatträger 7 ist mit einem unteren Trägerende 8 in einem unteren Lager 10 an einer unteren Behälterwand 6 drehbar gelagert. Ein oberes Trägerende 11 des Aggregatträgers 7 ragt durch die Öffnung 5 hindurch in einen Innenraum 12 des Doms 9.

Das obere Trägerende 11 umfaßt endseitig als Verlängerung des Aggregatträgers 7 eine ortsfeste Drehantriebswelle 13 eines Drehantriebs 14. Über diesen Drehantrieb 14 der Hubvorrichtung 1 ist der Aggregatträger 7 um dessen vertikale Längsachse 16 verdrehbar in einer bestimmten Drehstellung einstellbar und festlegbar. Dazu ist die ortsfeste Drehantriebswelle 13 durch eine gasdichte Buchse 17 als dichte Wellendurchführung durch eine obere Domwand 18 geführt.

Zur Betätigung des Drehantriebs 14 ist ein gelenkig mit dem äußeren Ende 19 der ortsfesten Drehantriebswelle 13 verbundener, aufschwenkbarer Stellhebel 20 als Betätigungseinrichtung vorgesehen. Dieser Stellhebel 20 ist über eine Gabelraste 21 als Arretiereinrichtung an der oberen Domwand 18 in einer bestimmten Drehstellung des Aggregatträgers 7 festlegbar.

Im Innenraum 12 des Doms 9 und damit innerhalb des Fermenterbehälters 3 ist eine Seiltrommel 24 mit horizontaler Seiltrommelwelle 26 über eine Konsole 25 mit dem Aggregatträger 7 fest verbunden. Ein auf die Seiltrommel 24 aufwickelbares Seil 27 ist mit einem freien Seilende 28 mit dem Tauchmotorrührgerät 2 zu dessen Höhenverstellung verbunden. Die Seillänge ist dabei so gewählt, daß das Tauchmotorrührgerät 2 maximal bis knapp vor die untere Behälterwand 6 abgesenkt werden kann.

Zur Höhenverstellung ist ferner ein Seiltrommelantrieb 30 vorgesehen, der einen an der Seiltrommel 24 angebrachten Elektromotor 31 umfaßt. Dieser Elektromotor 31 ist von außerhalb des Doms 9 über eine Steuereinheit 32 als Betätigungseinrichtung, die in der Fig. 1 nur schematisch als Kasten eingezeichnet ist, bevorzugt über einen Links-/Rechtslauf-Taster, ansteuerbar. Die Steuereinheit 32 ist über eine dichte Kabeldurchführung 33 durch die obere Domwand 18 mit dem Elektromotor 31 verbunden. Über die Steuereinheit 32 ist zudem auch der Antriebsmotor des Tauchmotorrührgeräts 2 steuerbar, was hier jedoch nicht dargestellt ist. Das Kabel 34 ist, wie dies aus der Fig. 1 ersichtlich ist, in einer Schleife geführt, um eine Verschwenkung des Aggregatträgers 7 um dessen Längsachse 17 mitmachen zu können.

Zur Höhenverstellung des Tauchmotorrührgeräts braucht somit lediglich der Links-/Rechtslauf-Taster der Steuereinheit 32 betätigt zu werden, woraufhin der Elektromotor 31 an der Seiltrommel 24 angesteuert wird. Der Elektromotor 31 bewegt die Seiltrommel 24 und damit das Seil 27 je nach eingestellter Laufrichtung so, daß das Tauchmotorrührgerät 2 über eine herkömmliche Rollenlagerung 22 entlang des Aggregatträgers 7 entweder nach unten in Richtung untere Behälterwand 6 oder in Richtung Dom 9 verfahren wird. Zur Verschwenkung des Aggregatträgers 7 um dessen Längsachse 16 wird der Stellhebel 20 aus der Gabelraste 21 herausbewegt, in eine gewünschte Rührerschwenkposition verschwenkt und anschließend wieder in eine versetzt angebrachte, der Schwenkposition zugeordnete Gabelraste eingerastet.

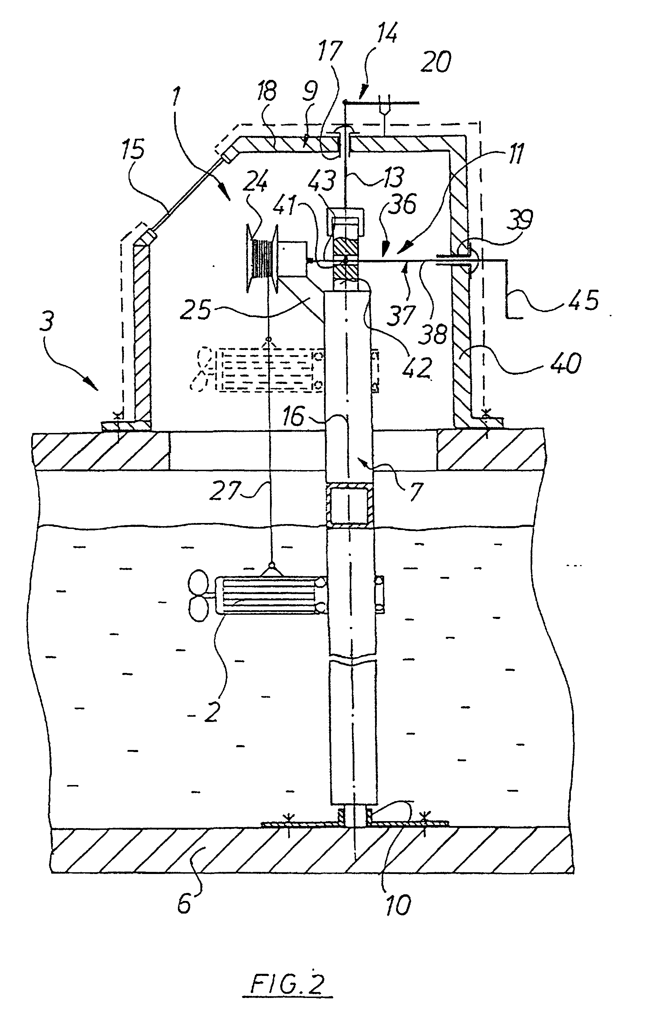

In der Fig. 2 ist eine Hubvorrichtung 1 dargestellt, die alternativ zur in der Fig. 1 dargestellten Ausführungsform einen Seiltrommelantrieb 36 mit einer Gelenkwelle 37 zeigt. Gleiche Teile in den Fig. 1 und 2 werden jeweils mit gleichen Bezugszeichen bezeichnet.

In der Fig. 2 ist eine ortsfeste Welle 38 der Gelenkwelle 37 des Seiltrommelantriebs 36 durch eine gasdichte Buchse als dichte Wellendurchführung 39 durch eine seitliche Domwand 40 geführt. Das Gelenk 41 der Gelenkwelle 37 dient als Mittel zur Umlenkung der Bewegungsübertragungsrichtung, wobei die Umlenkung in der Drehachse 16 des Aggregatträgers 7 erfolgt. Das Gelenk 41 ist z. B. ein Einfach-Wellengelenk, das in einem Gelenklager 42 am oberen Trägerende 11 geführt ist, wobei sich an das Gelenk 41 der Gelenkwelle 37 gegenüberliegend zur ortsfesten Welle 38 der Gelenkwelle 37 eine Seiltrommelwelle 43 der Gelenkwelle 37 anschließt. Diese Seiltrommelwelle 43 ist mit der Seiltrommel 24 drehübertragend verbunden. Zur Betätigung der Gelenkwelle 37 ist eine Kurbel 45 als Betätigungseinrichtung vorgesehen, die mit der ortsfesten Welle 38 des Seiltrommelantriebs 36 verbunden ist. Zur definierten Höhenverstellung des Tauchmotorrührgeräts 2 kann die Konsole 25 am oberen Trägerende 11 als Anschlag verwendet werden.

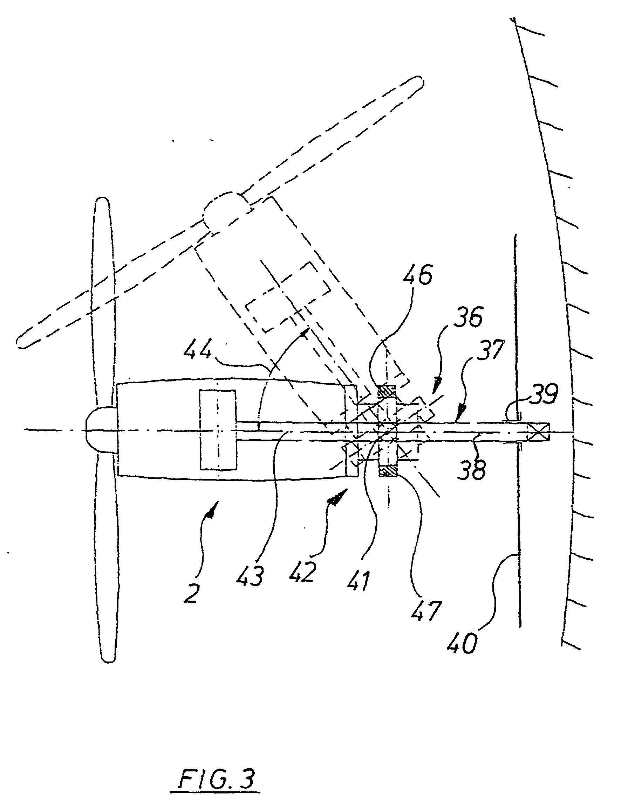

Zur Höhenverstellung kann hier über die Kurbel 45 eine Drehbewegung über die Gelenkwelle 37 auf die Seiltrommel 24 übertragen werden. Je nach der Drehrichtung kann dabei das Tauchmotorrührgerät 2 gehoben bzw. abgesenkt werden. Im Falle einer Verschwenkung des Aggregatträgers 7 um dessen Längsachse 16 mittels des Drehantriebs 14 kann die Seiltrommelwelle 43 in einer Ebene senkrecht zur vertikalen Längsachse 16 relativ zu der ortsfesten Welle 38 mitverschwenkt werden, wie dies in der Fig. 3 schematisch dargestellt ist. Die maximale Verschwenkung entsprechend Pfeil 44 des Tauchmotorrührgeräts 2 ist in der Darstellung der Fig. 3 strichliert eingezeichnet, wobei im Bereich des Gelenklagers 42 jeweils links und rechts von dem Gelenk 41 zwei Anschläge 46, 47 vorgesehen sind, die den maximalen Verschwenkgrad des Tauchmotorrührgeräts 2 vorgeben.

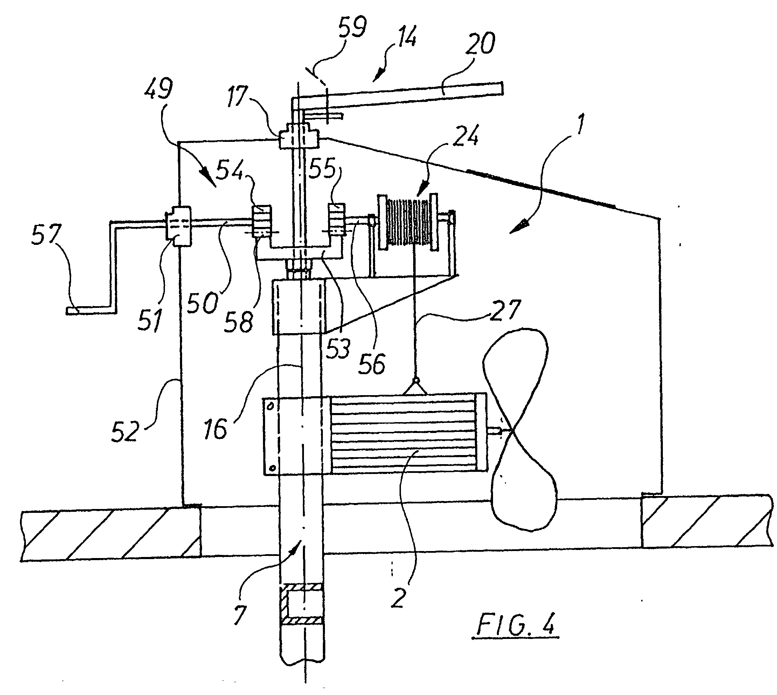

In der Fig. 4 ist eine Hubvorrichtung 1 dargestellt, bei der eine weitere alternative Ausführungsform eines Seiltrommelantriebs 49 dargestellt ist. Eine ortsfeste Welle 50 ist hier durch eine gasdichte Buchse 51 als dichte Wellendurchführung durch eine seitliche Domwand 52 geführt. Die ortsfeste Welle 50 weist endseitig ein geradverzahntes Zahnrad 54 auf, das mit einem geradverzahnten Zahnring 58 eines Tellerrads 53 des Seiltrommelantriebs 49 kämmt. Das Tellerrad 53 ist am Aggregatträger 7 frei drehbar angeordnet und steht gegenüberliegend zu der ortsfesten Welle 50 mit einem Zahnrad 55 in Eingriff, das endseitig an einer Seiltrommelwelle 56 angeordnet ist. Bei einer Betätigung einer mit der ortsfesten Welle 50 verbundenen Kurbel 57 wird über das Zahnrad 54 der ortsfesten Welle 50 die Drehbewegung auf das Tellerrad 53 übertragen. Das Tellerrad 53 überträgt anschließend die Bewegung auf das Zahnrad 55 der Seiltrommelwelle 56. Je nach gewählter Drehrichtung an der Kurbel kann somit das Tauchmotorrührgerät entweder nach oben in Richtung zu dem Dom 9 oder nach unten in Richtung zu einer unteren Behälterwand, die hier nicht dargestellt ist, verfahren werden. Zur Verschwenkung des Aggregatträgers 7 und damit des Tauchmotorrührgeräts 2 um die vertikale Längsachse 16 des Aggregatträgers 7 durch den Drehantrieb 14, der hier über einen Arretierstift 59 in einer bestimmten Verdrehstellung festlegbar ist, wird der Stellhebel 20 entsprechend des gewünschten Verstellwegs verschwenkt. Durch die frei drehbare Anordnung des Tellerrads 53 am Aggregatträger 7 ist gewährleistet, daß der Seiltrommelantrieb 49 diese Verschwenkbewegung des Aggregatträgers 7 nicht blockiert und die Bewegungsübertragung von der ortsfesten Welle 50 in jeder Verschwenkstellung des Aggregatträgers 7 auf die Seiltrommelwelle 56 möglich ist.

Wie dies in der Darstellung der Fig. 4 am oberen Trägerende 11 strichliert angedeutet ist, kann der Aggregatträger 7 zweiteilig aus einem unteren Trägerteil und einem oberen Trägerteil mit einer Trennlinie im Bereich unterhalb der festen Anbindung der Seiltrommel 24 an den Aggregatträger 7 aufgebaut sein, d.h. die Seiltrommel 24 ist bevorzugt am oberen Trägerteil fest angebunden. Damit ist eine optimale Anpassung an die jeweils vorherrschenden Einbaubedingungen möglich.

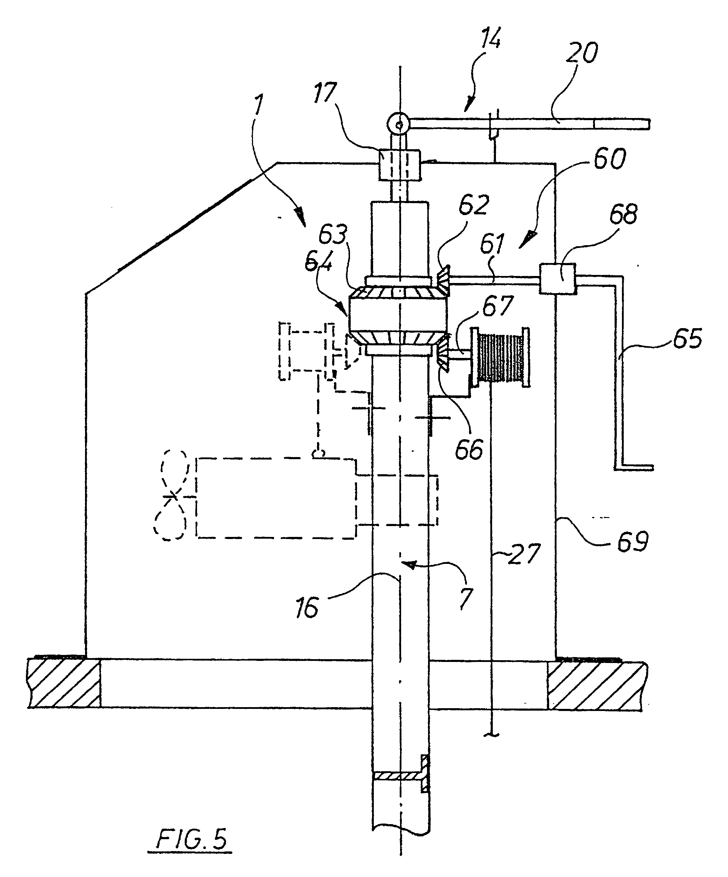

In der Fig. 5 ist eine Hubvorrichtung 1 dargestellt, bei der alternativ zu dem in der Fig. 4 dargestellten Seiltrommelantrieb 49 ein Seiltrommelantrieb 60 mit einer Kegelradanordnung dargestellt ist. Ein dominneres Ende einer ortsfesten Welle 61, die mittels einer Buchse 68 als Wellendurchführung gasdicht durch eine seitliche Domwand 69 geführt ist, umfaßt ein Kegelrad 62, das mit einem oberen Kegelradprofil 63 eines Kegelradrings 64 kämmt. Der Kegelradring 64 ist hier ebenfalls frei drehbar am Aggregatträger 7 angeordnet und überträgt die mittels einer Kurbel 65 über die ortsfeste Welle 61 und das Kegelrad 62 auf den Kegelradring 64 übertragene Bewegung auf ein Kegelrad 66 einer Seiltrommelwelle 67.

Je nach der gewählten Drehrichtung an der Kurbel 65 kann das Tauchmotorrührgerät 2 nach oben bzw. nach unten in der Höhe verstellt werden. Bei einer Verschwenkung des Aggregatträgers 7 um dessen Längsachse 16 und damit des Tauchmotorrührgeräts 2 bewirkt die freie, drehbare Anordnung des Kegelradrings 64 am Aggregatträger 7, daß die Verschwenkung nicht blockiert wird und die Bewegungsübertragung von der ortsfesten Welle 61 in jeder Verschwenkstellung des Aggregatträgers 7 auf die Seiltrommelwelle 67 möglich ist. Somit kann das Tauchmotorrührgerät 2, wenn es von der abgesenkten Stellung ausgehend in den Dominnenraum 12 bewegt wird, wie dies in der Fig. 5 strichliert eingezeichnet ist, so verschwenkt werden, daß das Tauchmotorrührgerät durch die Öffnung 5 hindurch in den Dominnenraum 12 bewegt werden kann.

Wie dies aus den Fig. 1, 2, 4 und 5 ersichtlich ist, kann der Aggregatträger 7 mit unterschiedlichen Profilquerschnitten ausgebildet sein, z. B. als Vierkantrohr, als U-Träger und als T-Träger. Mit einem derartigen Profilträger ist gewährleistet, daß das Tauchmotorrührgerät sich bei einer Verschwenkung des Aggregatträgers 7 mitdreht.

## Patentansprüche

1. Hubvorrichtung (1) für ein Aggregat, insbesondere für ein Tauchmotorrührgerät (2) in einem dichten Behälter (3), insbesondere in einem Fermenterbehälter einer Biogasanlage,
mit einem vertikal im Behälter (3) angeordneten Aggregatträger (7), der in oberen und unteren Lagern (17, 10) gelagert ist und an dem das Aggregat höhenverstellbar gehalten ist,
mit einer Höhenverstelleinrichtung als Seilwinde für das Aggregat, die eine mit dem Aggregatträger (7) fest verbundene Seiltrommel (24) mit horizontaler Seiltrommelwelle (26), ein einerseits mit dem Aggregat verbundenes und andererseits auf die Seiltrommel (24) aufwickelbares Seil (27), einen Seiltrommelantrieb (30; 36; 49; 60) und eine außerhalb des Behälters (3) angeordnete Betätigungseinrichtung (32; 45; 57; 65) für den Seiltrommelantrieb (30; 36; 49; 60) umfaßt,
**dadurch gekennzeichnet,**
**daß** die Seiltrommel (24) innerhalb im oberen Bereich des Behälters (3) angeordnet ist, so daß keine gasdichte Seildurchführung durch eine Behälterwand erforderlich ist, und
**daß** die außerhalb des Behälters (3) angeordnete Betätigungseinrichtung (32; 45; 57; 65) mit dem mittels einer dichten Durchführung (33; 39; 51; 68) durch eine Behälterwand geführten Seiltrommelantrieb (30; 36; 49; 60) verbunden ist.

2. Hubvorrichtung nach Anspruch 1, **dadurch gekennzeichnet, daß** der Behälter (3) einen Dom (9) umfaßt, der auf eine obere Behälterwand (4) dicht aufgesetzt ist, wobei ein oberer Bereich (11) des Aggregatträgers (7), an dem die Seiltrommel (24) angeordnet ist, in den Dom (9) ragt.

3. Hubvorrichtung nach Anspruch 1 oder Anspruch 2, **dadurch gekennzeichnet, daß** die Hubvorrichtung (1) eine Drehverstelleinrichtung für den Aggregatträger (7) mit einem Drehantrieb (14) umfaßt, mit der dieser um dessen vertikale Längsachse (16) verdrehbar in einer bestimmten Drehstellung einstellbar und festlegbar ist, wobei das Aggregat (2) an dem Aggregatträger (7) mitdrehend gehalten ist.

4. Hubvorrichtung nach Anspruch 3, **dadurch gekennzeichnet, daß** der Drehantrieb (14) der Drehverstelleinrichtung ein bevorzugt im oberen Bereich (11) des Aggregatträgers (7) innerhalb des Behälters (3) angebrachter Elektromotor ist, der eine Drehbewegung bevorzugt über eine Zahnradanordnung auf den Aggregatträger (7) überträgt und der von außerhalb des Behälters (3) über eine Steuereinheit als Betätigungseinrichtung, bevorzugt über einen Links-/Rechtslauf-Taster, ansteuerbar und über eine dichte Kabeldurchführung durch eine Behälterwand, bevorzugt eine Domwand, mit der Steuereinheit verbunden ist.

5. Hubvorrichtung nach Anspruch 3, **dadurch gekennzeichnet, daß** eine ortsfeste Drehantriebswelle (13) des Drehantriebs (14) des Aggregatträgers (7), bevorzugt als Verlängerung des Aggregatträgers (7), durch eine dichte Wellendurchführung (17) durch eine Behälterwand, bevorzugt eine Domwand (18), geführt ist.

6. Hubvorrichtung nach Anspruch 5, **dadurch gekennzeichnet, daß** eine Betätigungseinrichtung (20) für den Drehantrieb (14) ein gelenkig mit dem äußeren Ende (19) der ortsfesten Drehantriebswelle (13) verbundener, aufschwenkbarer Stellhebel ist, der in einer bestimmten Drehstellung über eine Arretiereinrichtung (21), bevorzugt eine Gabelraste, an einer Behälterwand, bevorzugt einer oberen Domwand (18), festlegbar ist.

7. Hubvorrichtung nach einem der Ansprüche 3 bis 6, **dadurch gekennzeichnet, daß** Anschläge zur Begrenzung einer Drehverstellung des Aggregatträgers (7) an dem Aggregatträger (7) und/oder einer Behälterwand, bevorzugt einer Domwand, angeordnet sind.

8. Hubvorrichtung nach einem der Ansprüche 1 bis 7, **dadurch gekennzeichnet, daß** der Seiltrommelantrieb (30) einen an der Seiltrommel (24) und damit innerhalb des Behälters (3) angebrachten Elektromotor (31) umfaßt, der von außerhalb des Behälters (3) über eine Steuereinheit (32) als Betätigungseinrichtung, bevorzugt über einen Links-/Rechtslauf-Taster, ansteuerbar und über eine dichte Kabeldurchführung (33) durch eine Behälterwand, bevorzugt eine Domwand (18), mit der Steuereinheit (32) verbunden ist.

9. Hubvorrichtung nach einem der Ansprüche 1 bis 7, **dadurch gekennzeichnet,**
**daß** eine ortsfeste Welle (38; 50; 61) des Seiltrommelantriebs (36; 49; 60) durch eine dichte Wellendurchführung (39; 51; 68) durch eine Behälterwand, bevorzugt eine seitliche Domwand (40; 53; 69), geführt ist,
**daß** der Seiltrommelantrieb (36; 49; 60) Mittel zur Umlenkung der Bewegungsübertragungsrichtung umfaßt, wobei die Umlenkung in der Drehachse (16) des Aggregatträgers (7) erfolgt, und
**daß** die Mittel zur Umlenkung der Bewegungsübertragungsrichtung einerseits mit der ortsfesten Welle (38; 50; 61) und andererseits mit der Seiltrommelwelle (43; 56; 67) verbunden sind.

10. Hubvorrichtung nach Anspruch 9, **dadurch gekennzeichnet, daß** die Mittel zur Umlenkung der Bewegungsübertragungsrichtung eine Gelenkwelle (37) umfassen, deren wenigstens ein Gelenk (41) in der Drehachse (16) des Aggregatträgers (7) liegt und deren beidseitige Wellenteile die ortsfeste Welle (38) und die Seiltrommelwelle (43) sind.

11. Hubvorrichtung nach Anspruch 9, **dadurch gekennzeichnet, daß** die Mittel zur Umlenkung der Bewegungsübertragungsrichtung einen Profilring (53; 64), bevorzugt einen geradverzahnten oder kegelradverzahnten Zahnring umfassen, der frei drehbar am Aggregatträger (7) angeordnet ist und der mit je einem zugeordneten Eingriffsprofil (54, 55; 62, 66) an der ortsfesten Welle (50; 61) und der Seiltrommelwelle (56; 67) in Eingriff steht.

12. Hubvorrichtung nach einem der Ansprüche 9 bis 11, **dadurch gekennzeichnet, daß** die Betätigungseinrichtung eine Kurbel (45; 57; 65) ist, die mit der ortsfesten Welle (38; 50; 61) des Seiltrommelantriebs (56; 67) verbunden ist.

13. Hubvorrichtung nach einem der Ansprüche 1 bis 12, **dadurch gekennzeichnet, daß** an dem Aggregatträger (7) wenigstens ein oberer Anschlag zur definierten Höhenverstellung des Aggregats (2) mit dem Seiltrommelantrieb (30; 36; 49; 60) angeordnet ist.

14. Hubvorrichtung nach einem der Ansprüche 1 bis 13, **dadurch gekennzeichnet, daß** der Aggregatträger (7) mit einem oberen Trägerende (11) oder einer gegebenenfalls mit dem oberen Trägerende verbundenen Drehantriebswelle (13) in einer oberen Behälterwand, bevorzugt einer oberen Domwand (18) und mit einem unteren Trägerende (8) in einer unteren Behälterwand (6) dicht gelagert ist.

15. Hubvorrichtung nach einem der Ansprüche 1 bis 14, **dadurch gekennzeichnet, daß** der Aggregatträger (7) zweiteilig aus einem unteren Trägerteil und einem oberen Trägerteil mit einer Trennlinie im Bereich unterhalb der festen Anbindung der Seiltrommel (24) an den Aggregatträger (7) aufgebaut ist, wobei das untere Trägerteil ein Profilträger ist und das obere Trägerteil wenigstens im Bereich einer Behälterwanddurchführung, bevorzugt einer Domwanddurchführung (17), eine ortsfeste Drehantriebswelle (13) eines Drehantriebs (14) bildet, die miteinander ggf. lösbar verbunden sind.

16. Hubvorrichtung nach einem der Ansprüche 1 bis 15, **dadurch gekennzeichnet, daß** der Aggregatträger (7) wenigstens teilweise als Profilträger mit offenem Profilquerschnitt ausgebildet ist.

## Claims

1. Hoisting apparatus (1) for an assembly, in particular for a submersible motorized stirrer (2) in a sealed vessel (3), in particular in a fermentation vessel of a biogas plant,
with an assembly support (7), which is arranged vertically in the vessel (3), is mounted in upper and lower bearings (17, 10) and on which the assembly is held in a height-adjustable manner,
with a height-adjusting device as a rope winch for the assembly, which device comprises a rope drum (24), which is firmly connected to the assembly support (7) and has a horizontal rope drum shaft (26), a rope (27), which at one end is connected to the assembly and at the other end can be wound onto the rope drum (24), a rope drum drive (30; 36; 49; 60) and an actuating device (32; 45; 57; 65), arranged outside the vessel (3), for the rope drum drive (30; 36; 49; 60),
**characterized**
**in that** the rope drum (24) is arranged within the upper region of the vessel (3), so that no gastight rope bushing through a vessel wall is required, and
**in that** the actuating device (32; 45; 57; 65) arranged outside the vessel (3) is connected to the rope drum drive (30; 36; 49; 60) led through a vessel wall by means of a sealed bushing (33; 39; 51; 68).

2. Hoisting apparatus according to Claim 1, **characterized in that** the vessel (3) comprises a dome (9) which is fitted in a sealed manner onto an upper vessel wall (4), an upper region (11) of the assembly support (7) on which the rope drum (24) is arranged projecting into the dome (9).

3. Hoisting apparatus according to Claim 1 or Claim 2, **characterized in that** the hoisting apparatus (1) comprises a rotational adjusting device for the assembly support (7) with a rotary drive (14), with which the latter can be set and fixed in a specific rotational position rotatably about its vertical longitudinal axis (16), the assembly (2) being held in a co-rotating manner on the assembly support (7).

4. Hoisting apparatus according to Claim 3, **characterized in that** the rotary drive (14) of the rotational adjusting device is an electric motor which is preferably fitted in the upper region (11) of the assembly support (7) within the vessel (3), transmits a rotational movement to the assembly support (7), preferably via a gearwheel arrangement, and can be activated from outside the vessel (3) by means of a control unit as an actuating device, preferably by means of a clockwise/anticlockwise-running button, and is connected to the control unit via a sealed cable bushing through a vessel wall, preferably a dome wall.

5. Hoisting apparatus according to Claim 3, **characterized in that** a fixed-in-place rotary drive shaft (13) of the rotary drive (14) of the assembly support (7) is led, preferably as an extension of the assembly support (7), through a sealed shaft bushing (17) through a vessel wall, preferably a dome wall (18).

6. Hoisting apparatus according to Claim 5, **characterized in that** an actuating device (20) for the rotary drive (14) is an adjusting lever which can be swung up, is connected in an articulated manner to the outer end (19) of the fixed-in-place rotary drive shaft (13) and can be fixed in a specific rotational position by means of an arresting device (21), preferably a fork rest, on a vessel wall, preferably an upper dome wall (18).

7. Hoisting apparatus according to one of Claims 3 to 6, **characterized in that** stops for limiting a rotational adjustment of the assembly support (7) are arranged on the assembly support (7) and/or a vessel wall, preferably a dome wall.

8. Hoisting apparatus according to one of Claims 1 to 7, **characterized in that** the rope drum drive (30) comprises an electric motor (31) which is fitted on the rope drum (24), and consequently inside the vessel (3), can be activated from outside the vessel (3) by means of a control unit (32) as an actuating device, preferably by means of a clockwise/anticlockwise-running button, and is connected to the control unit (32) via a sealed cable bushing (33) through a vessel wall, preferably a dome wall (18).

9. Hoisting apparatus according to one of Claims 1 to 7, **characterized**
**in that** a fixed-in-place shaft (38; 50; 61) of the rope drum drive (36; 49; 60) is led through a sealed shaft bushing (39; 51; 68) through a vessel wall, preferably a lateral dome wall (40; 53; 69),
**in that** the rope drum drive (36; 49; 60) comprises means for deflecting the direction of movement transmission, the deflection taking place in the axis of rotation (16) of the assembly support (7), and
**in that** the means for deflecting the direction of movement transmission are connected at one end to the fixed-in-place shaft (38; 50; 61) and at the other end to the rope drum shaft (43; 56; 67).

10. Hoisting apparatus according to Claim 9, **characterized in that** the means for deflecting the direction of movement transmission comprise a cardan shaft (37), at least one joint (41) of which lies in the axis of rotation (16) of the assembly support (7) and the shaft parts of which on either side are the fixed-in-place shaft (38) and the rope drum shaft (43).

11. Hoisting apparatus according to Claim 9, **characterized in that** the means for deflecting the direction of movement transmission comprise a profiled ring (53; 64), preferably a spur-gear or bevel-gear toothed ring, which is arranged freely rotatably on the assembly support (7) and is in engagement by a respective assigned engagement profile (54, 55; 62, 66) on the fixed-in-place shaft (50; 61) and the rope drum shaft (56; 67).

12. Hoisting apparatus according to one of Claims 9 to 11, **characterized in that** the actuating device is a crank (45; 57; 65), which is connected to the fixed-in-place shaft (38; 50; 61) of the rope drum drive (56; 67).

13. Hoisting apparatus according to one of Claims 1 to 12, **characterized in that** at least one upper stop for the defined height adjustment of the assembly (2) with the rope drum drive (30; 36; 49; 60) is arranged on the assembly support (7).

14. Hoisting apparatus according to one of Claims 1 to 13, **characterized in that** the assembly support (7) is mounted in a sealed manner by an upper support end (11), or a rotary drive shaft (13) possibly connected to the upper support end, in an upper vessel wall, preferably an upper dome wall (18), and by a lower support end (8) in a lower vessel wall (6).

15. Hoisting apparatus according to one of Claims 1 to 14, **characterized in that** the assembly support (7) is constructed in two parts from a lower support part and an upper support part with a separating line in the region below the fixed connection of the rope drum (24) to the assembly support (7), the lower support part being a profiled support and the upper support part forming, at least in the region of a vessel wall bushing, preferably a dome wall bushing (17), a fixed-in-place rotary drive shaft (13) of a rotary drive (14), which parts are connected to one another, possibly releasably.

16. Hoisting apparatus according to one of Claims 1 to 15, **characterized in that** the assembly support (7) is formed at least partly as a profiled support with an open profile cross section.

## Revendications

1. Dispositif de soulèvement (1) pour un agrégat, en particulier pour un agitateur submersible motorisé (2) dans une cuve hermétique, en particulier dans une cuve de fermentateur d'une installation de production de bio-gaz,
avec un support agrégat (7) disposé verticalement dans la cuve (3), monté dans des paliers supérieurs et inférieurs (17, 10) et sur lequel agrégat est maintenu, de façon réglable en hauteur,
avec un dispositif de réglage en hauteur réalisé sous la forme d'un treuil à câbles pour l'agrégat, comprenant un tambour à câble (24) relié rigidement au support d'agrégat (7), avec un arbre à tambour à câble (26) horizontal, un câble (27) relié d'un côté à l'agrégat et susceptible d'être enroulé de l'autre côté sur le tambour à câble (24) ; un entraînement de tambour à câble (30 ; 36 ; 49 ; 60) et un dispositif d'actionnement (32 ; 45 ; 57 ; 65) disposés à l'extérieur de la cuve (3), pour l'entraînement de tambour à câble (30 ; 36 ; 49 ; 60),
**caractérisé en ce que**,
le tambour à câble (24) est disposé intérieurement dans la zone supérieure de la cuve (3) de manière qu'aucun passage de câbles étanche au gaz n'ait à devoir être effectué à travers une paroi de cuve, et
**en ce que** le dispositif d'actionnement ((32 ; 45 ; 57 ; 65) disposé à l'extérieur de la cuve (3) est relié à l'entraînement de tambour à câble (30 ; 36 ; 49 ; 60) guidé à travers une paroi de cuve au moyen d'un passage (33 ; 39 ; 51 ; 68) étanche.

2. Dispositif de soulèvement selon la revendication 1, **caractérisé en ce que** la cuve (3) comprend un dôme (9) posé de façon étanche sur une paroi supérieure de cuve (4), une zone supérieure (11) du support d'agrégat (7), sur laquelle est disposé le tambour à câble (24), pénétrant dans le dôme (9).

3. Dispositif de soulèvement selon la revendication 1 ou la revendication 2, **caractérisé en ce que** le dispositif de levage (1) comprend un dispositif de manoeuvre rotative pour le support d'agrégat (7) avec un entraînement rotatif (14), dispositif à l'aide duquel celui-ci peut être tourné autour de son axe longitudinal (16) vertical, en pouvant être réglé et fixé en une position de rotation déterminée, l'agrégat (2) étant maintenu sur le support d'agrégat (7), de façon assujettie en rotation.

4. Dispositif de soulèvement selon la revendication 3, **caractérisé en ce que** l'entraînement relatif (14) du dispositif de manoeuvre rotative est un moteur électrique, monté de préférence dans la zone supérieure (11) du support d'agrégat (7) à l'intérieur de la cuve (3), moteur électrique transmettant un mouvement rotatif, de préférence par l'intermédiaire d'un dispositif à roue dentée, au support d'agrégat (7) et pouvant être commandé depuis l'extérieur de la cuve (3), par l'intermédiaire d'une unité de commande réalisée sous la forme de dispositif d'actionnement, de préférence d'une touche de manoeuvre à gauche/à droite, et étant reliée à l'unité de commande par l'intermédiaire d'un passage pour câbles étanche traversant la paroi de cuve de préférence une paroi de dôme.

5. Dispositif de soulèvement selon la revendication 3, **caractérisé en ce qu'**un arbre d'entraînement rotatif (13) localement fixe de l'entraînement rotatif (14) du support d'agrégat (7) est guidé, de préférence en tant que prolongement du support d'agrégat (7), à travers un guidage pour arbre (17) étanche, traversant une paroi de cuve, de préférence une paroi de dôme (18).

6. Dispositif de soulèvement selon la revendication 5, **caractérisé en ce qu'**un dispositif d'actionnement (20) pour l'entraînement rotatif (14) est un levier de réglage pouvant être levé par pivotement, relié de façon articulée à l'extrémité extérieure (19) de l'arbre d'entraînement rotatif (13) localement fixe, levier de réglage pouvant être fixé dans une position de rotation déterminée, par l'intermédiaire d'un dispositif de blocage (21), de préférence un cliquet à fourche, sur une paroi de cuve, de préférence une paroi de dôme supérieure (18).

7. Dispositif de soulèvement selon l'une des revendications 3 à 6, **caractérisé en ce que** des butées servant à délimiter le déplacement rotatif du support d'agrégat (7) sont disposées sur le support d'agrégat (7) et/ou une paroi de cuve, de préférence une paroi de dôme.

8. Dispositif de soulèvement selon l'une des revendications 1 à 7, **caractérisé en ce que** l'entraînement de tambour à câble (30) comprend un moteur électrique (31) monté sur le tambour à câble (24) et, ainsi, à l'intérieur de la cuve (3), moteur électrique qui peut être commandé, depuis l'extérieur de la cuve (3), par une unité de commande (32) réalisée sous la forme de dispositif d'actionnement, de préférence une touche de manoeuvre à gauche/à droite et reliée à l'unité de commande (32) par un passage de câbles (33) étanche traversant une paroi de cuve, de préférence une paroi de dôme (18).

9. Dispositif de soulèvement selon l'une des revendications 1 à 7, **caractérisé**
**en ce qu'**un arbre (28 ; 50 ; 61) localement fixe de l'entraînement de tambour à câble (30 ; 49 ; 60) est guidé, au moyen d'une traversée d'arbre (39 ; 51 ; 68) étanche, à travers une paroi de cuve de préférence une paroi de dôme latérale (40 ; 53 ; 69),
**en ce que** l'entraînement de tambour à câble (36 ; 49 ; 60) comprend des moyens pour dévier la direction de transmission cinématique, la déviation se faisant dans l'axe de rotation (16) du support d'agrégat (7), et
**en ce que** les moyens de déviation du sens de transmission cinématique sont reliés, d'une part, à l'arbre (38 ; 50 ; 61) localement fixe et, d'autre part, à l'arbre de tambour à câble (43 ; 56 ; 67).

10. Dispositif de soulèvement selon la revendication 9, **caractérisé en ce que** les moyens de déviation de la direction de transmission cinématique comprennent un arbre articulé (37), dont au moins une articulation (41) est placée dans l'axe de rotation (16) du support d'agrégat (7) et dont les parties d'arbre situées de part et d'autre sont l'arbre (38) localement fixe et l'arbre de tambour à câble (43).

11. Dispositif de soulèvement selon la revendication 9, **caractérisé en ce que** les moyens de déviation de la direction de transmission cinématique comprennent une bague profilée (53 ; 64), de préférence une bague dentée, à denture droite ou à denture pour roue conique, montée sur le support d'agrégat (7) de façon à pouvoir tourner librement et s'engrenant avec un profil d'engrènement (54, 55 ; 62, 66) chaque fois associé à l'arbre (50 ; 61) localement fixe et à l'arbre de tambour à câble (56 ; 67).

12. Dispositif de soulèvement selon l'une des revendications 9 à 11, **caractérisé en ce que** le dispositif d'actionnement est une manivelle (45 ; 57 ; 65) reliée à l'arbre localement fixe (38 ; 50 ; 61) de l'entraînement de tambour à câble (56 ; 67).

13. Dispositif de soulèvement selon l'une des revendications 1 à 12, **caractérisé en ce que**, sur le support d'agrégat (7), est disposée au moins une butée supérieure, pour obtenir un déplacement en hauteur définie de l'agrégat (2) à l'aide de l'entraînement de tambour à câble (30 ; 36 ; 49 ; 60).

14. Dispositif de soulèvement selon l'une des revendications 1 à 13, **caractérisé en ce que** le support d'agrégat (7) est monté de façon étanche, par une extrémité de support supérieure (11) ou un arbre d'entraînement rotatif (13), le cas échéant relié à l'extrémité de support supérieur, dans une paroi de cuve supérieure, de préférence une paroi de dôme supérieure (18) et, par une extrémité de support inférieure (8), dans une paroi de cuve inférieure (6).

15. Dispositif de soulèvement selon l'une des revendications 1 à 14, **caractérisé en ce que** le support d'agrégat (7) est formé en deux parties, d'une partie de support inférieure et d'une partie de support supérieure, avec une ligne de séparation placée dans la zone au-dessous de la liaison fixe du tambour à câble (24) sur le support d'agrégat (7), la partie de support inférieure formant un support ou poutre profilée et la partie de support supérieure formant, au moins dans la zone d'un passage de paroi de cuve, de préférence d'un passage de paroi de dôme (17), un arbre d'entraînement rotatif (13) localement fixe d'un entraînement rotatif (14), qui sont reliés ensemble le cas échéant de façon désolidarisable.

16. Dispositif de soulèvement selon l'une des revendications 1 à 15, **caractérisé en ce que** le support d'agrégat (7) est réalisé au moins partiellement sous forme de support profilé à section transversale de profil ouvert.
